# EUROPEAN PATENT APPLICATION

(11) **EP 1 275 388 A1**
(43) Date of publication of application: **15.01.2003**
(21) Application number: 01904345.4
(22) Date of filing: 08.02.2001
(51) Int. Cl.: A61K 31/22, A61K 31/405, A61K 31/40, A61K 31/4409, A61K 31/366, A61K 31/505, A61P 43/00, A61P 29/00, A61P 19/02, A61P 13/10, A61P 13/12, A61P 11/00, A61P 11/06, A61P 9/04, A61P 9/10, A61P 25/16, A61P 25/28, A61P 31/04, A61P 31/12, A61P 37/02

(54) **TNF- ALPHA INHIBITORS**

(30) Priority: 10.02.2000 JP 2000038266
(71) Applicant: Takeda Chemical Industries, Ltd., Osaka-shi, Osaka 541-0045 (JP)
(72) Inventor: SUGIYAMA, Yasuo;, HYOGO 66, 6-0111 (JP); ODAKA, Hiroyuki;, Hyogo 651-1223; (JP); NARUO, Ken-ichi;, HYOGO 669-1535 (JP); FUNATSU, Masami, OSAKA 572-0019 (JP); IKEYA, Kazuaki;, Nara 636-0131; (JP); SUZUKI, Yoshiharu, OSAKA 565-0823 (JP)
(74) Representative: Wright, Robert Gordon McRae
(86) International application number: JP0100881
(87) International publication number: WO01058443

(57) **Abstract**

There is provided a TNF-α inhibitor comprising at least one compound selected from cerivastatin, atorvastatin, simvastatin, pravastatin, itavastatin and (+)-(3R, 5S)-7-[4-(4-fluorophenyl)-6-isopropyl-2-(N-methyl-N-methanesulfonylamino)pyrimidin-5-yl]-3,5-dihydroxy-6(E)-heptenoic acid, or a salt thereof, which is excellent in the preventing and treating effects to TNF-α-associated diseases such as inflammatory disease and has the sufficiently excellent nature as a medicine, such as absence of side effect.

## Description

### Field of the Invention

The present invention relates to a TNF-α inhibitor, useful as an agent for preventing or treating TNF-α-associated diseases, for example, inflammatory diseases, which contains at least one compound selected from cerivastatin, atorvastatin, simvastatin, pravastatin, itavastatin and (+)-(3R, 5S)-7-[4-(4-fluorophenyl)-6-isopropyl-2-(N-methyl-N-methanesulfonylamino)pyrimidin-5-yl]-3,5-dihydroxy-6(E)-heptenoic acid, or a salt thereof.

### Background Art

TNF (tumor necrosis factor)-α is thought to play an important role in various diseases. For example, in rheumatoid arthritis which is an inflammatory disease, it is thought that production of TNF-α is enhanced and this causes destruction of the joint tissue.

Development of a TNF-α inhibitor is desired which is excellent in the effect of preventing and treating inflammatory diseases, and has the sufficiently excellent nature as a medicine such as absence of the side effect.

### SUMMARY OF THE INVENTION

The present invention relates to:
(1) a TNF-α inhibitor, which comprises at least one compound selected from cerivastatin ((+)-(3R, 5S, 6E)-7-[4-(4-fluorophenyl)-2,6-diisopropyl-5-methoxymethyl-3-pyridyl]-3,5-dihydroxy-6-heptenoic acid), atorvastatin ((βR, δR)-2-(4-fluorophenyl)-β, δ-dihydroxy-5-isopropyl-3-phenyl-4-(phenylcarbamoyl)pyrrole-1-heptanoic acid), simvastatin (1,2,3,7,8,8a-hexahydro-3,7-dimethyl-8-[2-(tetrahydro-4-hydroxy-2-oxo-2H-pyran-2-yl)ethyl]-1-naphthalenyl 2,2-dimethylbutanoate), pravastatin (1,2,6,7,8,8a-hexahydro-β, δ,6-trihydroxy-2-methyl-8-(2-methyl-1-oxobutoxy)-1-naphthaleneheptanoic acid), itavastatin (bis{(3R, 5S, 6E)-7-[2-cyclopropyl-4-(4-fluorophenyl)-3-quinolyl]-3,5-dihydroxy-6-heptenoic acid) and (+)-(3R, 5S)-7-[4-(4-fluorophenyl)-6-isopropyl-2-(N-methyl-N-methanesulfonylamino)pyrimidin-5-yl]-3,5-dihydroxy-6(E)-heptenoic acid, or a salt thereof,
(2) the TNF-α inhibitor described in the (1), wherein the compound is cerivastatin,
(3) the TNF-α inhibitor described in the (1), wherein the compound is atorvastatin,
(4) the TNF-α inhibitor described in the (1), which is an agent for preventing and treating TNF-α-associated diseases,
(5) the TNF-α inhibitor described in the (4), which is an anti-inflammatory agent,
(6) a method for inhibiting TNF-α, characterized by administering an effective amount of at least one compound selected from cerivastatin, atorvastatin, simvastatin, pravastatin, itavastatin and (+)-(3R, 5S)-7-[4-(4-fluorophenyl)-6-isopropyl-2-(N-methyl-N-methanesulphonylamino)pyrimidin-5-yl]-3,5-dihydroxy-6(E)-heptenoic acid, or a salt thereof,
(7) use of a compound selected from cerivastatin, atorvastatin, simvastatin, pravastatin, itavastatin and (+)-(3R, 5S)-7-[4-(4-fluorophenyl)-6-isopropyl-2-(N-methyl-N-methanesulfonylamino)pyrimidin-5-yl]-3,5-dihydroxy-6(E)-heptenoic acid, or a salt thereof, for preparing a TNF-α inhibitor, and
(8) a method for inhibiting TNF-α, characterized by administering at least one compound selected from cerivastatin, atorvastatin, simvastatin, pravastatin, itavastatin and (+)-(3R, 5S)-7-[4-(4-fluorophenyl)-6-isopropyl-2-(N-methyl-N-methanesulfonylamino)pyrimidin-5-yl]-3,5-dihydroxy-6(E)-heptenoic acid, or a salt thereof, at 2.5-1000µg/kg/day.

Cerivastatin, atorvastatin, simvastatin, pravastatin, itavastatin and (+)-(3R, 5S)-7-[4-(4-fluorophenyl)-6-isopropyl-2-(N-methyl-N-methanesulfonylamino)pyrimidin-5-yl]-3,5-dihydroxy-6(E)-heptenoic acid, are a commercially available compound, or can be obtained by the known method. For example, (+)-(3R, 5S)-7-[4-(4-fluorophenyl)-6-isopropyl-2-(N-methyl-N-methanesulfonylamino)pyrimidin-5-yl]-3,5-dihydroxy-6(E)-heptenoic acid, can be produced by a method described in EP-521471.

Examples of a salt of cerivastatin, atorvastatin, simvastatin, pravastatin, itavastatin and (+)-(3R, 5S)-7-[4-(4-fluorophenyl)-6-isopropyl-2-(N-methyl-N-methanesulfonylamino)pyrimidin-5-yl]-3,5-dihydroxy-6(E)-heptenoic acid include pharmaceutically acceptable salt, for example, salts with an inorganic base, salts with an organic base, salts with an inorganic acid, salts with an organic acid and salts with a basic or acidic amino acid, etc.

Preferable examples of salts with an inorganic base include salts with an alkali metal such as sodium and potassium, etc., an alkaline earth metal such as calcium and magnesium, etc., and aluminum and ammonium, etc.

Preferable examples of salts with an organic base include salts with trimethylamine, triethylamine, pyridine, picoline, ethanolamine, diethanolamine, triethanolamine, dicyclohexylamine and N,N-dibenzylethylenediamine, etc.

Preferable examples of salts with an inorganic acid include salts with hydrochloric acid, hydrobromic acid, nitric acid, sulfuric acid and phosphoric acid.

Preferable examples of salts with an organic acid include salts with formic acid, acetic acid, trifluoroacetic acid, fumaric acid, oxalic acid, tartaric acid, maleic acid, citric acid, succinic acid, malic acid, methanesulfonic acid, benzenesulfonic acid and p-toluenesulfonic acid, etc.

Preferable examples of salts with a basic amino acid include salts with arginine, lysine and ornithine, etc., and preferable examples of salts with acidic amino acid include salts with aspartic acid and glutamic acid, etc.

The TNF-α inhibitor of the present invention has the excellent TNF-α inhibitory activity, and is safely used as an agent for preventing and treating TNF-α-associated diseases in a mammal (for example, human being, mouse, rat, rabbit, dog, cat, cow, horse, pig, monkey and the like). The TNF-α-associated disease (induced by TNF-α) herein refers to a disease which is developed due to the presence of TNF-α and is treated through the TNF-α inhibiting effect. Examples of such diseases include inflammatory diseases [for example, diabetic complication such as retinopathy, nephrosis, neuropathy, great vessel disorder and the like; arthritis such as rheumatoid arthritis, osteoarthritis, rheumatoid myelitis, gouty arthritis, osteomyelitis and the like; lumbago; gout; inflammation after operation and trauma; remission of swelling; neuralgia; pharyngitis; cystitis; pneumonia; atopic dermatitis; inflammatory bowel disease such as Crohn's disease, ulcerarive colitis and the like; meningitis; inflammatory ocular disease; inflammatory pulmonary disease such as pneumonia, silicosis, pulmonary sarcoidosis, pulmonary tuberculosis and the like], curculatory system diseases (for example, angina pectoris, myocardial infarction, congestive heart failure, disseminated intravascular coagulation and the like), diabetic nephropathy, asthma, allergic disease, chronic obstractive pulmonary disease, systemic lupus erythematosus, Crohn's disease, autoimmune hemolytic anemia, psoriasis, nervous degenerative diseases (for example, Alzheimer disease, Parkinson's disease, amyotrophic lateral sclerosis, AIDS encephalopathy and the like), central nervous disorder (for example, cerebrovascular disorders such as cerebral hemorrhage and cerebral infarction, head trauma, spinal damage, cerebral edema, multiple scleroma and the like), toxemia (for example, sepsis, septic shock, endotoxin shock, gram negative sepsis, toxin shock syndrome and the like), Addison's disease, Creutzfeldt-Jakob disease, virus infective disease (for example, virus infective disease such as cytomegalovirus, influenzavirus, herpesvirus and the like), rejection response upon transplantation and dialytic hypotension.

The TNF-α inhibitor of the present invention may be used alone for treatment, or may be used together with other pharmaceutical ingredients including other lipid lowering drug or cholesterol lowering drug, myocardiac protecting drug, coronary disease treating drug, diabetes treating drug, thyroid gland disfunction treating drug, nephrosis syndrome treating drug or chronic renal insufficiency treating drug. In this case, these compounds are preferably administered parenteral or as an oral preparation, or may be administered as a rectal preparation in the form of a suppository, if necessary.

Examples of possible combinatorial ingredients include the following drugs.

These compounds may be used in a combination with:
diabetes treating drugs: kinedak, benfill, humulin, euglucon, glimicron, daonil, novoline, monotard, insulins, glucobay, dimelin, rastinon, basilcon, deamelin S, iszilins;
hypothyroidism treating drugs: dry thyroid gland (thyreoid), levothyroxine sodium (thyradin S), liothyronidine sodium (thyronine, thyromin); nephrotic syndrome treating drugs: usually, in the steroid therapy adopted as the first selection, prednisolone (predonine), prednisolone sodium succinate, methylprednisolone sodium succinate (solu-medrol), betamethasone (rinderon) and the like are used. In addition, in the anti-coaguration therapy, anti-platelet drugs such as dipyridamole (bersantin), dilazep dihydrochloride (comelian), tyropidine, chrobidgrel, Xa inhibitory agent and the like are used;
chronic renal insufficiency treating drugs: diuretic [for example, furosemide (lasix), bumetanide (lunetoron), azosemide (diart)], depressor (for example, ACE inhibitory drug, (enalapril maleate (renivace)) and Ca antagonizing drug (manidipine) and α receptor blocking drug or the like;
   and, upon administration, they can be used preferably by oral administration.

Further, the TNF-α inhibitors of the present invention are suitable for preventing and treating thrombus formation. Upon this, they can be used alone or in combination with the following known treating drugs, preferably by oral administration:
thrombus formation preventing and treating drugs: blood coagulation inhibiting drugs [for example, heparin sodium, heparin calcium, warfarin potassium (warfarin), Xa inhibiting drug], thrombolytic drug [for example, tPA, urokinase], anti-platelet drug [for example, aspirin, sulfinpyrazoro (anturan), dipyridamole (persantin), ticlopidine (panaldine) , cilostazol (pletaal), GPIIb/IIIa antagonizing drug (ReoPro)]
coronary vasodilator: nifedipine, diltiazem, nicoradil, nitrous acid agent
cardiac muscle protecting drug: cardiac ATP-K mouth drug, endothelin antagonizing drug, urotensin antagonizing drug.

The TNF-α inhibitor of the present invention may be used orally or parenterally, by injection, drip, inhalation, rectal or topical administration, and it can be used as it is or as a preparation for a pharmaceutical composition (for example, powders, granules, tablets, pills, capsules, injections, syrups, emulsions, elixirs, suspensions and solutions). For example, compounds as an active ingredient used in the present invention and the aforementioned compounds may be combined, and mixed or used together, and may be used by mixing with a pharmaceutically acceptable carrier (such as adjuvant, excipient, additive and/or diluent), if necessary.

The TNF-α inhibitor of the present invention can be formed into a preparation according to means normally used in preparation. Such the preparation can be usually prepared by mixing/kneading an active ingredient with an additive such as an excipient, a diluent, a carrier and the like. As used herein, parenieral method includes subcutaneous injection, intravenous injection, intramuscular injection, intraperitoneal injection and a drip method. A dispensing agent for injection, for example, aqueous suspensions or oily suspensions for aseptic injection can be prepared using a suitable dispersing agent or a wetting agent and a suspending agent by the method known in the art. The dispensing agent for aseptic injection may be a solution or a suspension, which can be aseptically injected, in a diluent or a solvent which is non-toxic and can be parenterally administered, such as an aqueous solution. Examples of the acceptable vehicle or solvent which can be used, include water, Ringer's solution and isotonic brine. Further, an aceptic nonvolatile oil is usually used as a solvent or a suspending solvent. For this purpose, any nonvolatile oil or fatty acid can be used, natural or synthetic or semi-synthetic fatty oil or fatty acid, and natural or synthetic or semi-synthetic mono-, di- or triglycerides may be contained.

Suppository for rectal administration can be prepared by mixing the drug with a suitable non-stimulating excipient, for example, excipients which are solid at a normal temperature but liquid at a temperature. of an intestinal tract, melts in rectum and releases a drug, such as cacao butter and polyethylene glycols.

Solid dosage forms for oral administration include powders, granules, tablets, pills and capsules. Preparations of such the dosage forms can be prepared by mixing and/or kneading an active ingredient compound with at least one additive, for example, sucrose, lactose, cellulose sugar, mannitol (D-mannitol), multitol, dextran, starches (for example, corn starch), microcrystalline cellulose, agar, alginates, chitins, chitosans, pectins, tragacanth gums, gums arabic, gelatins, collagens, caseine, albumin, synthetic or semi-synthetic polymers or glycerides. Such the dosage forms can contain further additives as usual, such as inert diluent, lubricant such as magnesium stearate, preservatives such as parabens and sorbic acid, antioxidant such as ascorbic acid, α-tocopherol and cysteine, disintegrating agent (for example, sodium crosscarmelose), binding agent (for example, hydroxypropylcellulose), tackifier, buffer agent, sweetener, flavor and perfume. Tablets and pills may be prepared by further subjecting to enteric coating. Examples of liquid preparations for oral administration include pharmaceutically acceptable emulsions, syrups, elixirs, suspensions and solutions, and they may contain an inert diluent normally used in the art, for example, water and additives if necessary. These oral liquid preparations can be prepared by mixing an active ingredient compound with an inert diluent and other additives if necessary according to the conventional method.

It is suitable that the preparations of the present invention contain an active ingredient compound at an amount of usually 0.01-99% by weight, preferably 0.1-90% by weight, more preferably 0.5-50% by weight depending upon the dosage form.

A dose is determined depending upon age, weight, general health condition, sex, diet, administration time, administration method, elimination rate, combination of drugs, degree of symptom of disease for which a patient is undergoing therapy, and considering those or other factors.

A dose per day of the TNF-α inhibitor of the present invention containing at least one selected from cerivasratin, atorvastatin, simvastatin, pravastatin, itavastatin and (+)-(3R, 5S)-7-[4-(4-fluorophenyl)-6-isopropyl-2-(N-methyl-N-methanesulfonylamino)pyrimidin-5-yl]-3,5-dihydroxy-6(E)-heptenoic acid, or a salt thereof, is different depending on the condition and the weight of the patient, a kind of a compound, an administration route and the like. For example, when used as an agent for preventing and treating hyperlipemia, a dose per day for an adult (weighing about 60kg) is about 1-500mg, preferably about 10-200mg in terms of an active ingredient in the case of an oral preparation, and about 0.1-100mg, preferably about 1-50mg, usually about 1-20mg in terms of an active ingredient in the case of a parenteral preparation. More specifically, as an oral preparation, the dose of cerivastatin is 0.15-0.30mg, the dose of atorvastatin is 10-60mg, the dose of simvastatin is 5-10mg, the dose of pravastatin is 10-20mg, the dose of itavastatin is 1-4mg, and the dose of (+)-(3R, 5S)-7-[4-(4-fluorophenyl)-6-isopropyl-2-(N-methyl-N-methanesulfonylamino)pyrimidin-5-yl]-3,5-dihydroxy-6(E)-heptenoic acid is 1-40mg. In this range, no toxicity is observed.

In addition, when used as a TNF-α inhibitor, a dose per day for an adult (weighing about 60kg) is about 0.15-60mg (2.5-1000µg/kg/day), preferably about 0.15-30mg (2.5-500µg/kg/day) in terms of an active ingredient in the case of an oral preparation, and is about 0.1-30mg (1.6-500µg/kg/day), preferably about 0.1-20mg (1.6-333µg/kg/day), usually about 0.1 (1.6-166µg/kg/day) in terms of an active ingredient in the case of an parenteral preparation. In this range, no toxicity is observed. Here, examples of the TNF-α inhibitor include those used for preventing and treating hyperlipemia at a dose per day for an adult (weighing about 60kg) of less than 20mg, inter alia, those used for preventing and treating hyperlipemia at a dose of less than 10mg, preferably those used for preventing and treating hyperlipemia at a dose of less than 5mg, more preferably those used for preventing and treating hyperlipemia at a dose of less than 1mg.

The TNF-α inhibiting effects by the compound of the present invention can be easily observed by the test, which is normally performed, as shown by the method known in the publication (The Journal of Pharmacology and Experimental Therapeutics, 291 (2), 680-687, (1999)).

### BEST MODE FOR CARRYING OUT THE INVENTION

The present invention will be explained more specifically by way of Reference Examples, but they do not limit the present invention.

### Examples

### Reference Example 1

20.5g of cerivastatin, 16160g of lactose and 540g of calcium carboxymethylcellulose (calcium carmelose) were placed in a fluidizing granulating drier (manufactured by Pawrek), pre-heated, and 7500g of an aqueous solution obtained by dissolving 450g of hydroxypropylcellulose is sprayed thereto to obtain granule powders. 16820g of the resulting granule powders are passed through a cutter mill (manufactured by Showakagaku-kikaikosakusyo) to obtain particle size-regulated powders, 513g of calcium carmelose and 57g of magnesium stearate are converted into mixed powders using a tumbling mixer (manufactured by Showakagakukikaikosakusyo), 16800g of the mixed powders are compressed with a compressing machine (manufactured by Kikusui-seisakusho) to obtain 140,000 tablets of the following composition containing 0.15mg of cerivastatin per tablet.

| Composition per tablet (unit:mg): | | |
|---|---|---|
| 1) | Cerivastatin | 0.15 |
| 2) | Lactose | 109.25 |
| 3) | Calcium carmelose | 7.2 |
| 4) | Hydroxypropylcellulose | 3.0 |
| 5) | Magnesium stearate | 0.4 |
| | Total | 120.0 |

### Reference Example 2

According to the same manner as that in Reference Example 1, 140,000 tablets of the following composition containing 33.1mg of atorvastatin per tablet are obtained.

| Composition per tablet (unit:mg): | | |
|---|---|---|
| 1) | Atorvastatin | 33.06 |
| 2) | Lactose | 76.34 |
| 3) | Calcium carmelose | 7.2 |
| 4) | Hydroxypropylcellulose | 3.0 |
| 5) | Magnesium stearate | 0.4 |
| | Total | 120.0 |

### Reference Example 3

According to the same manner as that in Reference Example 2, 140,000 tablets of the following composition containing 0.3mg of cerivastatin per tablet are obtained.

| Composition per tablet (unit:mg): | | |
|---|---|---|
| 1) | Cerivastatin | 0.3 |
| 2) | Lactose | 109.1 |
| 3) | Calcium carmelose | 7.2 |
| 4) | Hydroxypropylcellulose | 3.0 |
| 5) | Magnesium stearate | 0.4 |
| | Total | 120.0 |

### Experimental Example 1

A method for measuring the TNF-α inhibiting activity (in vitro)

The measurement is performed according to the method known in the publication (The Journal of Pharmacology and Experimental Therapeutics, 291(2), 680-687, (1999)).

That is, peripheral blood of rat, mouse, dog and human being is heparin-drawn, washed with several times using a culturing medium such as RPMI1640 medium by a centrifugation method to obtain peripheral mononuclear leukocyte. The resulting peripheral mononuclear leukocyte is suspended again in a culturing medium containing bovine fetal serum to a suitable cell concentration, and cultured at 37°C using a culturing instrument such as a flat bottom 96-well culturing plate. Upon this, a cell culture without a compound added is prepared. 1-24 hours after initiation of culturing, a culturing supernatant is taken. The TNF-α inhibiting activity of a compound can be measured by measuring an amount of TNF-α contained in the culturing supernatant by an ELISA method or a bioassay method.

The present measurement can be also performed using an established cell line producing TNF-α such as THP-1 cells.

### Experimental Example 2

A method for measuring the TNF-α inhibiting activity (in vivo)

The measurement is performed according to the method known in the publication (The Journal of Pharmacology and Experimental Therapeutics, 291(2), 680-687, (1999)).

That is, a compound is administered to an experimental animal such as rat, mouse and dog by any one method of oral, subcutaneous, intraperitoneal and intravenous administrations. After 30 minutes-5 hours, a group to which a suitable amount of lipopolysaccharide is administered intraperitoneally or intravenously and a group to which no lipopolysaccharide is administered are prepared. Further, after 30 minutes-3 hours, the plasma is taken by a suitable method, an amount of TNF-α contained in the plasma is measured by an ELISA method or a bioassay method, whereby, the TNF-α inhibiting activity of a compound can be measured.

### Industrial applicability

The TNF-α inhibitor of the present invention has the excellent TNF-α inhibitory activity, and useful as an agent for preventing and treating TNF-α-associated diseases, for example, inflammatory disease.

## Claims

1. A TNF-α inhibitor, which comprises at least one compound selected from cerivastatin, atorvastatin, simvastatin, pravastatin, itavastatin and (+)-(3R, 5S)-7-[4-(4-fluorophenyl)-6-isopropyl-2-(N-methyl-N-methanesulfonylamino)pyrimidin-5-yl]-3,5-dihydroxy-6(E)-heptenoic acid, or a salt thereof.

2. The TNF-α inhibitor according to claim 1, wherein the compound is cerivastatin.

3. The TNF-α inhibitor according to claim 1, wherein the compound is atorvastatin.

4. The TNF-α inhibitor according to claim 1, which is an agent for preventing and treating TNF-α-associated diseases.

5. The TNF-α inhibitor according to claim 4, which is an anti-inflammatory agent.

6. A method for inhibiting TNF-α, **characterized by** administering an effective amount of at least one compound selected from cerivastatin, atorvastatin, simvastatin, pravastatin, itavastatin and (+)-(3R, 5S)-7-[4-(4-fluorophenyl)-6-isopropyl-2-(N-methyl-N-methanesulfonylamino)pyrimidin-5-yl]-3,5-dihydroxy-6(E)-heptenoic acid, or a salt thereof.

7. Use of a compound selected from cerivastatin, atorvastatin, simvastatin, pravastatin, itavastatin and (+)-(3R, 5S)-7-[4-(4-fluorophenyl)-6-isopropyl-2-(N-methyl-N-methanesulfonylamino)pyrimidin-5-yl]-3,5-dihydroxy-6(E)-heptenoic acid, or a salt thereof, for preparing a TNF-α inhibitor.

8. A method for inhibiting TNF-α, **characterized by** administering at least one compound selected from cerivastatin, atorvastatin, simvastatin, pravastatin, itavastatin and (+)-(3R, 5S)-7-[4-(4-fluorophenyl)-6-isopropyl-2-(N-methyl-N-methanesulfonylamino)pyrimidin-5-yl]-3,5-dihydroxy-6(E)-heptenoic acid, or a salt thereof, at 2.5-1000µg/kg/day.
